(19) Europäisches Patentamt
European Patent Office
Office européen des brevets

(11) Veröffentlichungsnummer: **0 330 020 B1**

(12) **EUROPÄISCHE PATENTSCHRIFT**

(45) Veröffentlichungstag der Patentschrift: **20.07.94**

(51) Int. Cl.$^{5}$: **C07D 285/12**, C07D 277/36, C07D 249/12, C07D 417/14, C07D 403/14, C08F 222/40, C08G 73/12

(21) Anmeldenummer: **89102234.5**

(22) Anmeldetag: **09.02.89**

Verbunden mit 89902042.4/0394361 (europäische Anmeldenummer/Veröffentlichungsnummer) durch Entscheidung vom 30.01.92.

Die Akte enthält technische Angaben, die nach dem Eingang der Anmeldung eingereicht wurden und die nicht in dieser Patentschrift enthalten sind.

# (54) Diamine und Bismaleinimide sowie daraus hergestellte Polyimide.

(30) Priorität: **26.02.88 AT 506/88**

(43) Veröffentlichungstag der Anmeldung:
**30.08.89 Patentblatt 89/35**

(45) Bekanntmachung des Hinweises auf die Patenterteilung:
**20.07.94 Patentblatt 94/29**

(84) Benannte Vertragsstaaten:
**AT BE CH DE ES FR GB GR IT LI LU NL SE**

(56) Entgegenhaltungen:
**US-A- 3 380 964**

**CHEMICAL ABSTRACTS, Band 107, Nr. 26, 28. Dezember 1987, Seite 13, Zusammenfassung Nr. 237486d, Columbus, Ohio, US; & JP-A-62138472**

**CHEMICAL ABSTRACTS, Band 96, Nr. 4, 25. Januar 1982, Seite 5, Zusammenfassung Nr. 20532k, Columbus, Ohio, US; M.M. KOTON et al.: "Polyimides containing different heterocyclic units in the main chain",**

(73) Patentinhaber: **Chemie Linz Gesellschaft m.b.H.**
**St.Peter-Strasse 25**
**A-4021 Linz(AT)**

(72) Erfinder: **Horacek, Heinz**
**Bockgasse 43**
**A-4020 Linz(AT)**

(74) Vertreter: **Kunz, Ekkehard, Dr.**
**Chemie Linz GmbH**
**Abteilung Patente**
**St. Peter-Str. 25**
**A-4021 Linz (AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die Erfindung betrifft Diamine und Bismaleinimide, sowie daraus hergestellte, gegebenenfalls faserverstärkte Polyimide.

Polyimide auf Basis von Bismaleinimiden sind bereits aus der US-A-3 380 964 bekannt, ihre Herstellung ist durch Erhitzen der Bismaleinimide bei Temperaturen von 80 bis 400 ° C möglich, wobei unter anderem Ethylen-bis-maleinimid, Diphenylmethan-bis-maleinimid, Diphenylether-bis-maleinimid oder Diphenylsulfon-bis-maleinimid eingesetzt werden.

Ein wesentlicher Nachteil dieser bekannten Polyimide liegt jedoch darin, daß sie spröde, schwer schmelzbar und schwer löslich sind, sodaß sie nur schwierig und nur in begrenztem Umfang weiter verarbeitbar sind. Es lassen sich beispielsweise keine Filme oder Folien aus ihnen herstellen, sondern nur harte Laminate.

Die Aufgabe der Erfindung war es daher, Diamine und Bismaleinimide zu finden, die zu flexiblen, weniger spröden und warmverformbaren Polyimiden polymerisiert werden können, die in nachfolgenden Arbeitsgängen, beispielsweise bei der Imprägnierung von Fasergeweben oder Fasermatten bzw. durch Formpressen, leicht weiter verarbeitet werden können.

Die Lösung der Aufgabe wurde mit Hilfe von bestimmten Diaminen und Bismaleinimiden gefunden, die in ihrer Kette einen über Äther- bzw. Thioäthergruppen verbundenen Thiazol-, Thiadiazol- oder Triazolring enthalten, und aus denen verbesserte Polyimide hergestellt werden konnten.

Die Verwendung von Heterocyclen wie z. B. Pyridin, Oxadiazol oder Benzimidazol enthaltenden Diaminen zur Herstellung von Polyimiden auf Basis von aromatischen Tetracarbonsäuren ist beispielsweise aus JP-Kokai 1987-138,472 (C.A. 107: 237486d) bzw. aus Vysokomol. Soedin., Ser.A 1981,23(8)1736-42 (C.A. 96:20532 k) bekannt.

Gegenstand der vorliegenden Erfindung sind demnach Diamine und Bismaleinimide der allgemeinen Formel I,

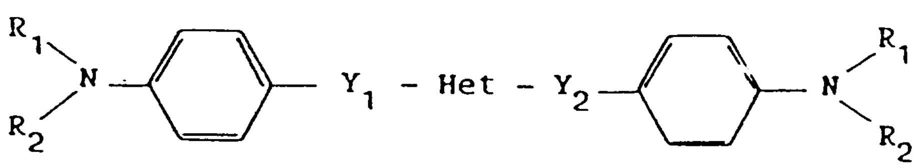

I

in der $R_1$ und $R_2$ ein H-Atom oder gemeinsam die Gruppe - OC - CH = CH - CO -, $Y_1$ und $Y_2$ unabhängig voneinander O oder S, und Het einen Thiazol- oder Thiadiazolring bedeuten. Bevorzugt sind Verbindungen, in denen $Y_1$ und $Y_2$ gleich sind.

Ein weiterer Gegenstand der Erfindung sind Verbindungen der Formel II,

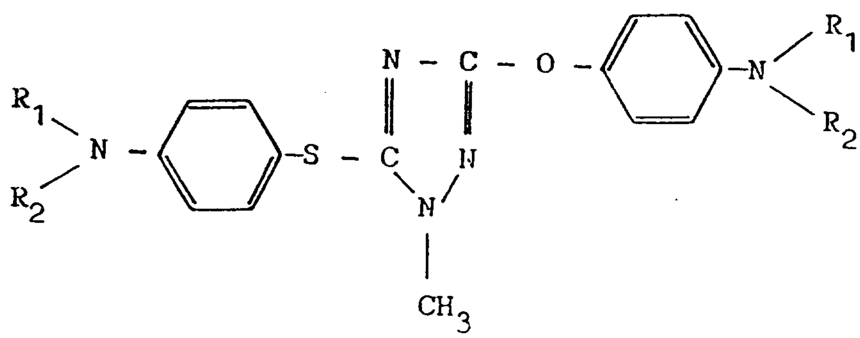

II

in der $R_1$ und $R_2$ ein H-Atom oder gemeinsam die Gruppe -OC-CH = CH-CO-bedeuten.

Für den Fall, daß $R_1$ und $R_2$ ein H-Atom bedeuten, stellen die erfindungsgemäßen chemischen Verbindungen aromatische Diamine, und für den Fall, daß $R_1$ und $R_2$ gemeinsam die Gruppe - OC - CH = CH - CO - bedeuten, aromatische Bismaleinimide dar.

Die Herstellung der erfindungsgemäßen Substanzen erfolgt durch Reaktion von Dimercapto- oder Dihydroxyheterocyclen, vorzugsweise in Form ihrer Alkalisalze, der allgemeinen Formel III oder IV:

(K) Na - $Y_1$ - Het - $Y_2$ - Na (K)    III

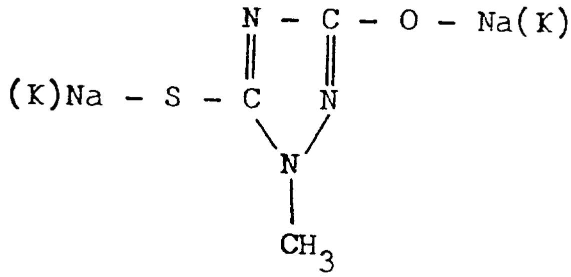

IV

in der $Y_1$, $Y_2$ und Het die oben angegebene Bedeutung haben, mit Chloranilin, wobei man unter Abspaltung von NaCl oder KCl das erfindungsgemäße Diamin der allgemeinen Formel I oder II erhält. Es ist auch möglich, die Verbindungen der allgemeinen Formel III oder IV mit p-Chlornitrobenzol umzusetzen, wobei man unter Abspaltung von NaCl oder KCl vorerst die entsprechenden Dinitroverbindungen erhält, welche anschließend mittels Wasserstoffs zum erfindungsgemäßen Diamin der allgemeinen Formel I oder II reduziert werden. Die erfindungsgemäßen Bismaleinimide der allgemeinen Formel I oder II erhält man durch Umsetzen der erfindungsgemäßen Diamine der allgemeinen Formel I oder II mit Maleinsäure oder deren Derivaten, beispielsweise mit Maleinsäureanhydrid.

Die Herstellung der Ausgangsverbindungen gemäß Formel III oder IV erfolgt nach bekannten Verfahren, beispielsweise in Anlehnung an die DE-C-958 650, DE-A-2 360 623, gemäß J.V. Metzger "Thiazole and its Derivatives", Chemistry of Heterocyclic Compounds Vol 34 Part 1 (1979) p. 215, J. Wiley, New York oder gemäß Frank F. Cesark (Northwestern Univ., Evanston, Ill). Univ. Microfilms (Ann Arbor, Mich.), Order No. 61-5299, 101 pp.; Dissertation Abstr. 22, 2192 (1962).

Die erfindungsgemäßen Diamine und Bismaleinimide können zur Herstellung von Polyimiden mit verbesserten Eigenschaften verwendet werden. Die Bismaleinimide können entweder für sich allein, oder gemeinsam mit den Diaminen zu Polyimiden polymerisiert werden.

Die aus den erfindungsgemäßen Diaminen und Bismaleinimiden hergestellten Polyimide sind ein weiterer Gegenstand der Erfindung. Die Polyimide bestehen aus sich wiederholenden monomeren Einheiten von Verbindungen gemäß Formel I oder II, wobei im Falle der Verwendung von Bismaleinimiden allein, die Polymerbindung über die Doppelbindungen der Maleinimidreste erfolgt, und im Falle der Verwendung von Bismaleinimiden und Diaminen außerdem eine Anlagerung der $NH_2$ -Gruppen des Diamins an die Doppelbindung des Maleinimidrestes erfolgt. Bevorzugt sind Polyimide, die aus 0 bis 0,8 Molen Diamin je 1 Mol Bismaleinimid aufgebaut sind, wobei ein molares Verhältnis von Diamin zu Bismaleinimid von 0,2 : 1 bis 0,6 : 1 besonders bevorzugt ist.

Zur Verbesserung der mechanischen Eigenschaften können die Polyimide für bestimmte Anwendungen vorteilhaft mit Fasern verstärkt werden, wobei sowohl Kurzfasern als auch Fasermatten, Fasergelege oder Gewebe zur Verstärkung eingesetzt werden können. Der Anteil an Verstärkungsfasern im Verbund liegt dabei üblicherweise bei etwa 20 - 70 Gew.%. Das Flächengewicht der Fasermatten liegt üblicherweise bei etwa 250 - 1.200 $g/m^2$, jenes von Geweben bei etwa 100 - 300 $g/m^2$.

Entsprechend den Ansprüchen an die mechanischen Eigenschaften können beispielsweise Glasfasern, Kohlefasern, Keramikfasern oder Aramidfasern, bzw. Mischungen aus diesen Fasern oder Hybridgelege bzw. Hybridgewebe eingesetzt werden. Kohlefasern oder Aramidfasern kommen vor allem dort zum Einsatz, wo insbesondere an Festigkeit, Steifigkeit und geringes spezifisches Gewicht der aus den Polyimiden hergestellten Teile besondere Anforderungen gestellt werden.

In vielen Fällen erweist es sich als besonders vorteilhaft, die Polyimide nicht vollständig auszuhärten, sodaß sie noch verformbar, schmelzbar und hitzehärtbar bleiben, und gegebenenfalls in einem nachfolgenden Heißpreßvorgang zu einem Fertigteil umgeformt werden können. Dies ist im Falle von Prepregs von Bedeutung. Prepregs bestehen bevorzugt aus mit Fasergeweben bzw. Fasergelegen verstärkten, nur teilweise ausgehärteten Polyimiden, sind lagerfähig und können auch noch nach Monaten zum gewünschten Fertigteil heißverpreßt werden.

Die Herstellung der erfindungsgemäßen Polyimide erfolgt beispielsweise durch Aufschmelzen des erfindungsgemäßen Bismaleinimids, gegebenenfalls gemeinsam mit dem erfindungsgemäßen Diamin, auf Temperaturen von 100 bis 300 °C, wobei ein Temperaturbereich von 120 bis 160 °C besonders bevorzugt ist. Die Ausgangsmaterialien werden vor und nach dem Aufschmelzen vorteilhafterweise gemahlen. Entsprechend der gewählten Temperatur und der Dauer des Aufschmelzens erfolgt eine mehr oder weniger vollständige Aushärtung des Polyimidharzes. Je nach verwendeten Einsatzstoffen kann eine weitgehende Aushärtung des Polyimidharzes beispielsweise bei einer Wärmebehandlung von etwa 80 Minuten bei etwa 120 °C, oder innerhalb von 15 Minuten bei etwa 160 °C erreicht werden. Bei nicht ausreichender Wärmebehandlung härtet das Polyimid nur teilweise aus, und kann in einem zu einem späteren Zeitpunkt nachfolgenden Verfahrensschritt endgültig zum gewünschten Fertigteil ausgehärtet werden.

Zur Herstellung von faserverstärkten Polyimidharzen können beispielsweise die Verstärkungsfasern mit dem Bismaleinimid und gegebenenfalls dem Diamin gemischt und anschließend unter Bildung des Polyimidharzes erhitzt und aufgeschmolzen werden. Es ist auch möglich, die Fasern mit dem bereits teilweise ausgehärteten Polyimidpulver zu mischen und gegebenenfalls zu verschmelzen. Die Herstellung faserverstärkter Laminate oder Prepregs gelingt beispielsweise dadurch, daß man ein Fasergelege oder Gewebe mit dem pulverförmigen Bismaleinimid und Diamin, oder dem bereits teilweise ausgehärteten Polyimidpulver imprägniert. Dabei können Lösungsmittel, beispielsweise Dimethylformamid (DMF) oder N-Methylpyrrolidon (NMP) verwendet werden, ihre Anwesenheit ist jedoch nicht unbedingt erforderlich.

Besonders vorteilhaft gestaltet sich die kontinuierliche Herstellung von faserverstärkten Laminaten, wobei man die pulverförmigen Komponenten beispielsweise auf das Band einer Doppelbandpresse dosiert und gleichzeitig eine oder mehrere Lagen der Faserverstärkung mit in die Presse einlaufen läßt. In der Doppelbandpresse erfolgt die Imprägnierung der Matten, Gelege oder Gewebe mit den aufgeschmolzenen Ausgangsstoffen, sowie die Bildung bzw. Aushärtung der Polyimide bei Drücken von 1 bis 20 bar und Temperaturen von 100 bis 300 °C. Die Fertigung von Polyimid-Prepregs erfolgt beispielsweise auf Imprägnieranlagen unter Einsatz von Geweben und Lösungen des erfindungsgemäßen Polyimidharzes in NMP. Die Konzentration der Lösungen liegt bei 20 - 60 Gew.%. Die Temperatur im Trockner, beispielsweise einem Trockenturm bzw. einem waagrechten Trockentunnel, beträgt 150 - 200 °C. Die nur teilweise ausgehärteten faserverstärkten Polyimid-Prepregs lassen sich in einem späteren Verarbeitungsschritt unter Druck- und Temperatureinwirkung zu einem Fertigteil verarbeiten, beispielsweise zu Leiterplatten, Elektrospulkernen, Teilen von Verbrennungsmotoren oder Geräteteilen für die Luft- und Raumfahrt.

Beispiel 1:

1.1. Herstellung des Diamins (2,5 Bis (4-Aminophenylthio)-1,3,4-thiadiazol)

Die Herstellung des Diamins erfolgte in Anlehnung an E. Müller, Houben-Weyl, Methoden der Organischen Chemie, Band IX (1955) p. 93 und Band XI/1 (1957) p. 341, G. Thieme, Stuttgart.

In einem 250 ml Dreihalskolben wurden 3 g Natriumhydroxid in 5 ml Wasser gelöst, 3,8 g (25 mmol 2,5-Dimercapto-1,3,4-thiadiazol zugegeben und auf 100 °C erhitzt. Die entstandene klare Lösung wurde 30 Minuten gerührt, worauf 79 g (50 mmol) 1-Chlor-4-Nitrobenzol, gelöst in 75 ml DMF, zugetropft wurden. Das Reaktionsgemisch wurde 4 Stunden unter Rückfluß gekocht, dann weitgehend eingedampft und abgekühlt. Der Rückstand wurde in Wasser aufgenommen, gerührt, abfiltriert und mit Wasser gewaschen. Der Filterrückstand wurde in Aceton gelöst, mit Wasser gefällt, filtriert und getrocknet. Man erhielt 8,8 g (80 % d. Th.) 2,5 Bis-(4-Nitrothiophenyl)-1,3,4-thiadiazol mit einem Schmelzpunkt von 210 °C und einem Schwefelgehalt von 24,5 Gew.%.

Die Nitroverbindung wurde in Toluol gelöst (5 %ige Lösung), 2,5 Gew.% eines Palladium-Kohlenstoffkatalysators (Fa. Heraeus, 5 % Pd auf C) zugesetzt und sodann bei 120 °C mittels Tauchrohres während 7 Stunden Wasserstoff eingeleitet, bis kein Wasserstoffverbrauch mehr erfolgte. Nach Abfiltration des Katalysators und Eindampfen der Lösung erhielt man 7,3 g (97 % d. Th.) 2,5 Bis-(4-Aminophenylthio)-1,3,4-thiadiazol).

Fp = 253 °C

Schwefel-Gehalt = 29 Gew.%

1.2. Herstellung des Bismaleinimids (2,5 Bis (4-maleinimidophenylthio)-1,3,4-thiadiazol)

332 g (1 Mol) des gemäß 1.1 hergestellten Diamins wurden in 500 g DMF gelöst und 196 g (2 Mol) Maleinsäureanhydrid, gelöst in 500 g DMF, bei -5 °C portionsweise zugegeben. Danach fügte man 300 g Essigsäureanhydrid und 15 g Na-Acetat (fest) zu, erwärmte auf 55 °C und hielt diese Temperatur während 1 Stunde. Dann ließ man das Reaktionsgemisch langsam in 5000 g Eiswasser einlaufen und rührte während 3 Stunden, wobei das Bismaleinimid ausfiel. Der Niederschlag wurde abfiltriert und mit 4000 g Wasser gewaschen, bis das Filtrat neutral war. Nach dem Trocknen wurden 454 g 2,5 Bis(4-maleinimidophenylthio)-1,3,4-thiadiazol (90 % d. Th.) erhalten.

Fp = 263 °C

1.3. Herstellung des Polyimids

Die gemäß 1.1 und 1.2 hergestellten Diamine und Bismaleinimide wurden im Molverhältnis 0,4 : 1

vermischt, auf eine Korngröße von unter 0,2 mm gemahlen und innerhalb von 5 Minuten bei 150 °C aufgeschmolzen. Das erhaltene Polyimid wurde abgekühlt und wieder auf eine Korngröße von unter 0,2 mm gemahlen.

24 kg/h des Polyimidpulvers wurden aus einem Schüttkasten auf das Band einer Doppelbandpresse dosiert und gleichzeitig ein Glasgewebe mit 296 g/m$^2$ Flächengewicht (Nr. 92626, Interglas) zugefahren. Die Temperatur in der Heizzone der Doppelbandpresse betrug 150 °C, der Preßdruck 5 bar, die Bandgeschwindigkeit lag bei 2 m/min. Nach dem Abkühlen unter Druck in einer anschließenden Kühlzone der Doppelbandpresse erhielt man eine 0,23 mm dicke, 1 m breite Prepregplatte. Die Eigenschaften der Platte nach dem Aushärten (10 Minuten bei 160 °C drucklos, 1 Stunde bei 180 °C und 20 bar Druck, 48 Stunden bei 200 °C drucklos) sind in Tabelle 1 zusammengestellt.

Beispiel 2:

2.1. Herstellung des Diamins (2-(4-Aminophenylthio)4-(4-Aminophenyloxo)-1,3-thiazol)

3 g Natriumhydroxid wurden in einem 250 ml Dreihalskolben in 5 ml Wasser gelöst und 3,3 g (25 mmol) Rhodanin zugegeben. Die erhaltene klare Lösung wurde auf 100 °C erwärmt und 6,25 g (50 mmol) p-Chloranilin, in 75 ml DMF gelöst, zugegeben, worauf man 4 Stunden unter Rückfluß kochte. Anschließend wurde unter Vakuum weitgehend eingedampft und der Rückstand in Wasser aufgenommen. Der Niederschlag wurde filtriert, in Aceton gelöst, mit Wasser gefällt, filtriert und getrocknet. Man erhielt 6,3 g 2-(4-Aminophenylthio)-4-(4-Aminophenyloxo)-1,3-thiazol (80 % d. Th.) mit 20,3 Gew.% Schwefelgehalt und einem Schmelzpunkt von 175 °C.

2.2. Herstellung des Bismaleinimids (2-(4-Maleinimidophenylthio)4-(4-Maleinimidophenyloxo)-1,3-thiazol)

315 g des gemäß 2.1 hergestellten Diamins (1 Mol) wurden in 500 g DMF gelöst und 196 g (2 Mol) Maleinsäureanhydrid, in 500 g DMF gelöst, bei -5 °C portionsweise zugegeben. Danach fügte man 300 g Essigsäureanhydrid und 15 g Natriumacetat (fest) zu, erwärmte das Reaktionsgemisch auf 55 °C und hielt eine Stunde bei dieser Temperatur. Danach ließ man das Reaktionsgemisch langsam in 5000 g Eiswasser zulaufen und rührte 3 Stunden, wobei das Bismaleinimid ausfiel. Der Niederschlag wurde abfiltriert und mit etwa 4000 g Wasser gewaschen, bis das Filtrat einen pH-Wert von 7 aufwies. Nach dem Trocknen erhielt man 438 g (90 % d. Th.) 2-(4-Maleinimidophenylthio)4-(4-Maleinimidophenyloxo)-1,3-thiazol mit einem Schmelzpunkt von 205 °C.

2.3. Herstellung des Polyimids

Die gemäß 2.1 und 2.2 hergestellten Diamine und Bismaleinimide wurden im Molverhältnis 0,4 : 1 gemeinsam während 5 Minuten bei 150 °C aufgeschmolzen. Die erkaltete Schmelze wurde auf eine Korngröße von unter 0,1 mm vermahlen und in NMP zu einer 50 Gew.%igen Lösung (Viskosität bei Raumtemperatur: 0,5 Pas) gelöst.

Ein 1 m breites Glasgewebe mit 296 g/m$^2$ Flächengewicht (Nr. 92626, Interglas) wurde auf einer kontinuierlichen Imprägnieranlage bei 23 °C mit dieser Lösung imprägniert. Die Spaltbreite der Rakel wurde so gewählt, daß nach dem Trocknen bei 150 °C während 8 Minuten im Trockenturm ein Prepreg mit einem Harzgehalt von 40 Gew.% erhalten wurde. Das Prepreg wurde anschließend während 15 Minuten bei 160 °C drucklos und während 1 Stunde bei 180 °C und 20 bar Druck gehärtet, sowie während 48 Stunden bei 200 °C drucklos getempert. Die Dicke des Laminats betrug 0,24 mm, das Flächengewicht 493 g/m$^2$. Die mechanischen Eigenschaften des Laminats sind in Tabelle 1 zusammengestellt.

Beispiel 3:

Die gemäß 1.1 und 1.2 hergestellten Diamine und Bismaleinimide wurden im Molverhältnis 0,4 : 1 gemeinsam während 5 Minuten bei 150 °C aufgeschmolzen. Die erkaltete Schmelze wurde auf eine Korngröße von unter 0,1 mm vermahlen und in NMP zu einer 50 Gew.%igen Lösung (Viskosität bei Raumtemperatur: 1 Pas) gelöst. Auf einer kontinuierlichen Imprägnieranlage wurde ein Gelege aus unidirektionalen (UD) Kohlefaserrovings (820 tex, Toho Rayon(R) HTA7 12000) mit 185 Strängen pro 1 m Breite mit der Polyimidlösung imprägniert. Die Spaltbreite der Rakel wurde so gewählt, daß nach dem Trocknen bei 150 °C während 8 Minuten im Trockenturm ein Prepreg mit einem Harzgehalt von 40 Gew.% erhalten wurde. Das Prepreg wurde anschließend während 15 Minuten bei 160 °C drucklos und während 1 Stunde bei 180 °C und 20 bar Druck gehärtet, sowie während 48 Stunden bei 200 °C drucklos getempert. Die Dicke des Laminats betrug 0,12 mm, das Flächengewicht 250 g/m$^2$. Die mechanischen Eigenschaften des Laminats sind in Tabelle I zusammengestellt.

Beispiel 4:

Analog zu Beispiel 3 wurde ein faserverstärktes Laminat hergestellt, wobei jedoch als Faserverstärkung statt der Kohlefaser ein Gelege aus unidirektionalem (UD) Kevlar 49 (Aramidfaser von Du Pont), das aus 950 Strängen zu je 158 tex pro 1 m Breite bestand, zur Imprägnierung mit der Polyimidlösung verwendet wurde.

Das ausgehärtete Laminat war 0,14 mm dick, das Flächengewicht lag bei 250 g/$m^2$. Die mechanischen Eigenschaften sind in Tabelle I zusammengestellt.

Beispiel 5:

Analog zu Beispiel 3 wurde ein faserverstärktes Laminat hergestellt, wobei jedoch als Faserverstärkung statt der UD-Kohlefaser ein unidirektionales (UD) Gelege aus einer Keramikfaser (Si-Ti-C-O-Faser, Tyranno$^{(R)}$ von Fa. Ube, Japan), das aus 1500 Strängen zu je 100 tex pro 1 m Breite bestand, zur Imprägnierung mit der Polyimidlösung verwendet wurde.

Das ausgehärtete Laminat war 0,12 mm dick, das Flächengewicht lag bei 250 g/$m^2$. Die mechanischen Eigenschaften des Laminats sind in Tabelle I zusammengestellt.

Beispiel 6:

Analog zu Beispiel 3 wurde ein faserverstärktes Laminat hergestellt, wobei jedoch als Faserverstärkung statt der Kohlefaser ein unidirektionaler Glasroving der Fa. Gevetex, BRD (EC 11-2400 K 247(50)) mit der Polyimidlösung imprägniert wurde. Pro 1 m Breite wurden 65 Stränge mit je 2400 tex verlegt.

Das ausgehärtete Laminat war 1,2 mm dick, das Flächengewicht lag bei 250 g/$m^2$. Die mechanischen Eigenschaften sind in Tabelle I zusammengestellt.

Beispiel 7:

7.1. Herstellung des Diamins (1-Methyl-3-(4-aminophenyloxo)-5(4-aminophenylthio)-1,2,4-triazol)

In einem 250 ml Dreihalskolben wurden 3 g NaOH in 5 ml Wasser gelöst und 2,33 g (25 mmol) 1 Methyl-3-hydroxy-5-mercapto-1,2,4-triazol (Herstellung gemäß DE-OS 23 60 623) zugegeben. Die erhaltene klare Lösung wurde auf 100 °C erwärmt, 6,25 g (50 mmol) p-Chloranilin, in 75 ml DMF gelöst, zugegeben und 4 h unter Rückfluß gekocht. Anschließend wurde unter Vakuum weitgehend eingedampft und der Rückstand in Wasser aufgenommen. Der Niederschlag wurde filtriet, in Aceton gelöst, mit Wasser gefällt, filtriert und getrocknet, wobei 6,33 g 1-Methyl-3-(4-aminophenyloxo-)5(4-aminophenylthio-)1,2,4-triazol (85 % d. Th.) erhalten wurden. Der Schwefelgehalt betrug 10,7 Gew.%, der Schmelzpunkt lag bei 180 °C.

7.2. Herstellung des Bismaleinimids

5,6 g Maleinsäureanhydrid wurden unter Rühren in 28,8 ml Toluol gelöst und innerhalb einer Stunde bei maximal 30 °C eine Lösung aus 6,9 g (23 mmol) des Diamins gemäß 7.1. in 15,8 ml Toluol und 4,8 ml Dimethylformamid (DMF) zudosiert, wobei eine Suspension der gelben Amidsäure gebildet wurde. Anschließend setzte man 0,45 g p-Toluolsulfonsäure zu und kochte das Reaktionsgemisch während 4 Stunden am Rückfluß bei 112 °C. Dabei wurden über einen Wasserabscheider insgesamt 0,8 ml Wasser abgeschieden, wonach eine klare Lösung vorlag.

Das Toluol wurde anschließend unter Vakuum abdestilliert, der Rückstand mit 11,4 ml Aceton verdünnt und bei 10 °C während 2 Stunden mit 28 ml einer 2 %igen wäßrigen Sodalösung versetzt. Anschließend rührte man 15 min, filtrierte den gebildeten Niederschlag ab, wusch 2 mal mit 14 ml Wasser und trocknete bei 60 °C im Vakuum. Es wurden 9,5 g des entsprechenden Bismaleinimids erhalten (90 % d. Th.)

7.3. Herstellung des Polyimids

Die gemäß 7.1. und 7.2. hergestellten Diamine und Bismaleinimide wurden im Molverhältnis 0,4 : 1 während 5 Minuten bei 150 °C versintert. Die erkaltete Schmelze wurde auf eine Korngröße von kleiner als 0,1 mm vermahlen und in NMP zu einer 50 %igen Lösung gelöst.

Anschließend wurde mit dieser Lösung analog zu 2.3. ein glasfaserverstärktes Laminat mit einer Dicke von 0,24 mm und einem Flächengewicht von 493 g/$m^2$ hergestellt. Die mechanischen Eigenschaften des Laminats sind in Tabelle 1 zusammengestellt.

Beispiel 8:

Die gemäß 7.1 und 7.2 hergestellten Diamine und Bismaleinimide wurden im Molverhältnis 0,4 : 1 gemeinsam während 5 Minuten bei 150 °C aufgeschmolzen. Die erkaltete Schmelze wurde auf eine Korngröße von unter 0,1 mm vermahlen und in NMP zu einer 50 Gew.%igen Lösung (Viskosität bei Raumtemperatur: 1 Pas) gelöst. Auf einer kontinuierlichen Imprägnieranlage wurde ein Gelege aus unidirektionalen (UD) Kohlefaserrovings (820 tex, Toho Rayon(R) HTA7 12000) mit 185 Strängen pro 1 m Breite mit der Polyimidlösung imprägniert. Die Spaltbreite der Rakel wurde so gewählt, daß nach dem Trocknen bei 150 °C während 8 Minuten im Trockenturm ein Prepreg mit einem Harzgehalt von 40 Gew.% erhalten wurde. Das Prepreg wurde anschließend während 15 Minuten bei 160 °C drucklos und während 1 Stunde bei 180 °C und 20 bar Druck gehärtet, sowie während 48 Stunden bei 200 °C drucklos getempert. Die Dicke des Laminats betrug 0,12 mm, das Flächengewicht 250 $g/m^2$. Die mechanischen Eigenschaften des Laminats sind in Tabelle I zusammengestellt.

Beispiel 9:

Analog zu Beispiel 8 wurde ein faserverstärktes Laminat hergestellt, wobei jedoch als Faserverstärkung statt der Kohlefaser ein Gelege aus unidirektionalem (UD) Kevlar 49 (Aramidfaser von Du Pont), das aus 950 Strängen zu je 158 tex pro 1 m Breite bestand, zur Imprägnierung mit der Polyimidlösung verwendet wurde.

Das ausgehärtete Laminat war 0,14 mm dick, das Flächengewicht lag bei 250 $g/m^2$. Die mechanischen Eigenschaften sind in Tabelle I zusammengestellt.

Beispiel 10:

10.1. Herstellung des Diamins (3,5-Bis(4-aminophenylthio)-1,2,4-thiadiazol)

In einem 250 ml Dreihalskolben wurden 3 g NaOH in 5 ml Wasser gelöst, 3,8 g (25 mmol) 3,5-Dimercapto-1,2,4-thiadiazol (Herstellung analog zu US 3 899 502, bzw. Z. Anorgan. Allgem. Chemie Vol. 486 (1982), Seite 111 - 115) zugegeben und auf 100 °C erhitzt. Zu der klaren Lösung wurden 79 g (50 mmol) 1-Chlor-4-Nitrobenzol, gelöst in 75 ml DMF, zudosiert. Nach 4 Stunden Kochen am Rückfluß wurde eingedampft und der Rückstand in Wasser aufgenommen, gewaschen und abfiltriert. Nach dem Umkristallisieren aus einem Aceton-Wassergemisch erhielt man 9 g (92 % d. Th.) 3,5-Bis(4-nitrophenylthio)1,2,4-thiadiazol).

Die erhaltene Nitroverbindung wurde zu einer 5 %igen Lösung in Toluol gelöst, mit 2,5 Gew.% Palladium-Kohlenstoffkatalysator versetzt und bei 120 °C hydriert, bis kein Wasserstoff mehr verbraucht wurde. Nach Abfiltration des Katalysators und Eindampfen der Lösung erhielt man 8 g 3,5-Bis(4-aminophenylthio)thiadiazol mit einem Schwefelgehalt von 29 Gew.%.

10.2. Herstellung des Bismaleinimids

332 g (1 Mol) des gemäß 10.1. hergestellten Diamins wurden analog zu 1.2. mit 2 Mol Maleinsäureanhydrid umgesetzt, wobei 460 g 3,5-Bis (4-maleinimidophenylthio)-1,2,4-thiadiazol mit einem Schmelzpunkt von 180 °C erhalten wurden.

10.3. Herstellung des Polyimids

Die gemäß 10.1. und 10.2. hergestellten Diamine und Bismaleinimide wurden im Molverhältnis 0,4 : 1 in NMP zu einer 50 %igen Lösung gelöst, in einem Rohrreaktor (Ullmann, Enzyklopädie der Techn. Chemie, Band 3, Seite 350, Verlag Chemie 1973) während 5 min auf 150 °C erhitzt und in Wasser gefällt. Das ausfallende Polymere wurde abfiltriert und das NMP rückgeführt. Das erhaltene Polymer wurde in NMP zu einer 50 %igen Lösung gelöst.

Anschließend wurde mit dieser Lösung analog zu 2.3. ein glasfaserverstärktes Laminat hergestellt, die Eigenschaften sind in Tabelle 1 zusammengestellt.

Beispiel 11:

Die gemäß 10.1. und 10.2. hergestellten Diamine und Bismaleinimide wurden im Molverhältnis 0,4 : 1 in NMP zu einer 50 %igen Lösung gelöst, in einem Rohrreaktor während 5 min auf 150 °C erhitzt und in Wasser gefällt. Das ausfallende Polymere wurde in NMP zu einer 50 %igen Lösung gelöst.

Auf einer kontinuierlichen Imprägnieranlage wurde ein Gelege aus unidirektionalen (UD) Kohlefaserrovings (820 tex, Toho Rayon(R) HTA7 12000) mit 185 Strängen pro 1 m Breite mit dieser Polymerlösung imprägniert. Die Spaltbreite der Rakel wurde so gewählt, daß nach dem Trocknen bei 150 °C während 8

Minuten im Trockenturm ein Prepreg mit einem Harzgehalt von 40 Gew.% erhalten wurde. Das Prepreg wurde anschließend während 15 Minuten bei 160 °C drucklos und während 1 Stunde bei 180 °C und 20 bar Druck gehärtet, sowie während 48 Stunden bei 200 °C drucklos getempert. Die Dicke des Laminats betrug 0,12 mm, das Flächengewicht 250 g/m². Die mechanischen Eigenschaften des Laminats sind in Tabelle 1 zusammengestellt.

Beispiel 12:

Analog zu Beispiel 11 wurde ein faserverstärktes Laminat hergestellt, wobei jedoch als Faserverstärkung statt der Kohlefaser ein Gelege aus unidirektionalem (UD) Kevlar 49 (Aramidfaser von Du Pont), das aus 950 Strängen zu je 158 tex pro 1 m Breite bestand, zur Imprägnierung mit der Polyimidlösung verwendet wurde.

Das ausgehärtete Laminat war 0,14 mm dick, das Flächengewicht lag bei 250 g/m². Die mechanischen Eigenschaften sind in Tabelle I zusammengestellt.

Tabelle I

| Polyimidlaminate mit 60 Gew.% Verstärkungsfasern | | | | | | |
|---|---|---|---|---|---|---|
| Zugfestigkeit* (N/mm²) | | Dehnung* (%) | E-Modul* (N/mm²) | Schlagzähigkeit** (J/m) | Dichte (g/cm³) | Verstärkung |
| 1 | 600 | 6 | 23 000 | 1 000 | 2,1 | Glasgewebe |
| 2 | 650 | 5 | 24 000 | 1 000 | 2,1 | Glasgewebe |
| 3 | 1700 | 1,5 | 1 30 000 | - | 1,7 | C-Faser UD |
| 4 | 1300 | 3 | 75 000 | - | 1,4 | Aramid UD |
| 5 | 1300 | 0,8 | 90 000 | - | 2,1 | Keramik UD |
| 6 | 1100 | 4 | 51 000 | - | 2,1 | Glas UD |
| 7 | 500 | 5 | 28 000 | 900 | 2,2 | Glasgewebe |
| 8 | 1700 | 1,5 | 120 000 | - | 1,6 | C-Faser UD |
| 9 | 1350 | 2,5 | 76 000 | - | 1,4 | Aramid UD |
| 10 | 550 | 5 | 25 000 | 1100 | 2,2 | Glasgewebe |
| 11 | 1300 | 1,3 | 140 000 | - | 1,6 | C-Faser UD |
| 12 | 1200 | 2,5 | 70 000 | - | 1,4 | Aramid UD |

* gemäß Norm EN 61

** gemäß Izod

## Patentansprüche

**1.** Diamine und Bismaleinimide der allgemeinen Formel I,

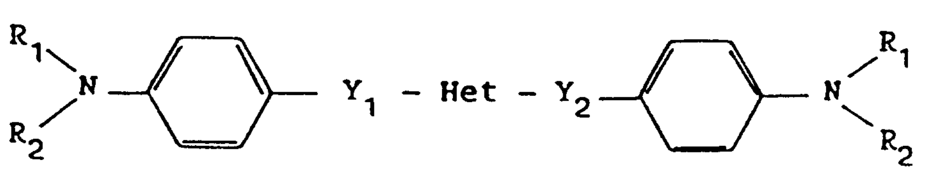

I

in der $R_1$ und $R_2$ ein H-Atom oder gemeinsam die Gruppe -OC-CH = CH-CO-, $Y_1$ und $Y_2$ unabhängig voneinander O oder S, und Het einen Thiazol- oder Thiadiazolring bedeuten.

**2.** Verbindungen der Formel II,

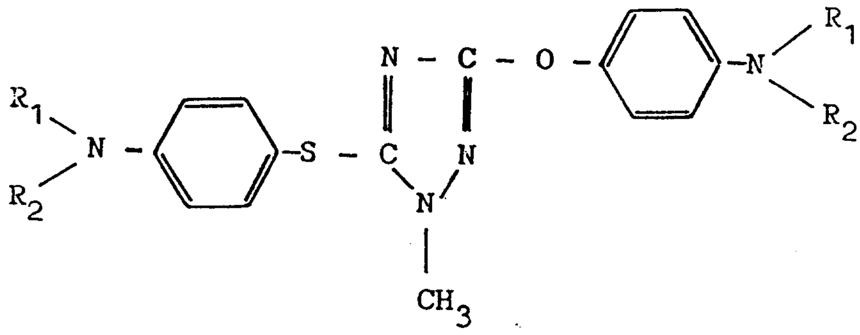

II

in der $R_1$ und $R_2$ ein H-Atom oder gemeinsam die Gruppe -OC-CH = CH-CO-bedeuten.

**3.** Diamine und Bismaleinimide gemäß Anspruch 1, dadurch gekennzeichnet, daß $Y_1$ und $Y_2$ gleich sind.

**4.** Verfahren zur Herstellung von Diaminen oder Bismaleinimiden gemäß einem der Ansprüche 1 bis 3, dadurch gekennzeichnet, daß man eine Verbindung der allgemeinen Formel III oder IV:

(K) Na - $Y_1$ - Het - $Y_2$ - Na (K) III

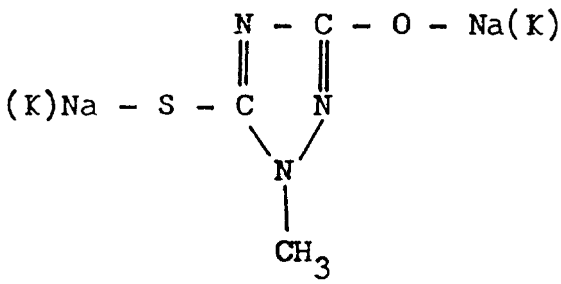

IV

in der $Y_1$, $Y_2$ und Het die oben angegebene Bedeutung haben, entweder mit p-Chloranilin oder mit p-Chlornitrobenzol umsetzt, wobei bei Verwendung von p-Chlornitrobenzol die beiden Nitrogruppen des erhaltenen Reaktionsproduktes anschließend zu Aminogruppen reduziert werden, worauf man die so erhaltenen Diamine der allgemeinen Formel I oder II, gegebenenfalls mit Maleinsäure oder deren Derivaten zu Bismaleinimiden der allgemeinen Formel I oder II umsetzt.

**5.** Verwendung von Bismaleinimiden der allgemeinen Formel I oder II, gegebenenfalls gemeinsam mit Diaminen der allgemeinen Formel I oder II gemäß einem der Ansprüche 1 bis 3, zur Herstellung von Polymaleinimiden.

**6.** Polyimide, bestehend aus sich wiederholenden Einheiten, die sich von Bismaleinimiden der allgemeinen Formel I oder II und gegebenenfalls Diaminen der allgemeinen Formel I oder II gemäß einem der Ansprüche 1 bis 3 ableiten, wobei das molare Verhältnis von Diamin zu Bismaleinimid 0 : 1 bis 0,8 : 1 beträgt.

**7.** Polyimide gemäß Anspruch 6, dadurch gekennzeichnet, daß sie Diamine und Bismaleinimide in einem molaren Verhältnis von 0,2 : 1 bis 0,6 : 1 enthalten.

**8.** Polyimide gemäß Anspruch 6 oder 7, dadurch gekennzeichnet, daß sie nicht vollständig ausgehärtet sind.

**9.** Polyimide gemäß einem der Ansprüche 6 bis 8, dadurch gekennzeichnet, daß sie mit Fasern verstärkt sind.

**10.** Verfahren zur Herstellung von Polyimiden gemäß einem der Ansprüche 6 bis 9, dadurch gekennzeichnet, daß man Bismaleinimide der allgemeinen Formel I oder II mit oder ohne Diamine der allgemeinen Formel I oder II bei 100 bis 300 °C verschmilzt, wobei ein Polyimidharz entsteht, das gegebenenfalls erst in einem weiteren Verfahrensschritt vollständig ausgehärtet wird, und daß man gegebenenfalls die Monomerkomponenten oder das nicht vollständig ausgehärtete Polyimidharz mit Verstärkungsfasern in Kontakt bringt.

## Claims

**1.** Diamines or bismaleimides of the general formula I

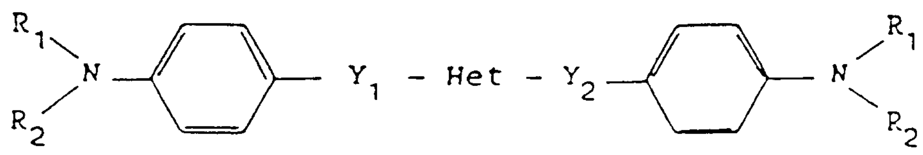

I

in which $R_1$ and $R_2$ denote an H atom or together denote the group -OC-CH = CH-CO-, $Y_1$ and $Y_2$ independently of one another denote O or S and Het denotes a thiazole or thiadiazole ring.

**2.** Compounds of the formula II

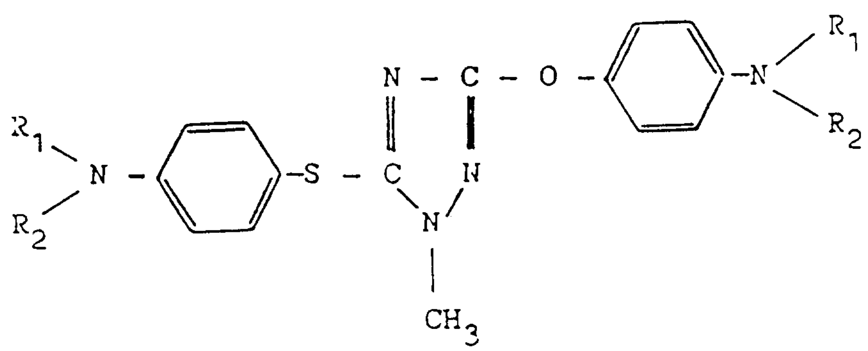

II

in which $R_1$ and $R_2$ denote an H atom or together denote the group -OC-CH = CH-CO-.

**3.** Diamines or bismaleimides according to Claim 1, characterised in that $Y_1$ and $Y_2$ are identical.

**4.** Process for the preparation of diamines or bismaleimides according to one of Claims 1 to 3, characterised in that a compound of the general formula III or IV:

(K)Na - $Y_1$ - Het - $Y_2$ - Na(K) III

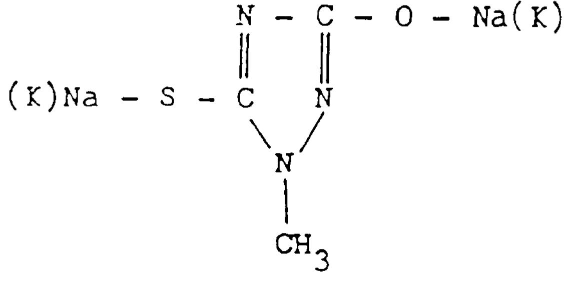

IV

in which $Y_1$, $Y_2$ and Het have the abovementioned meaning, is reacted with either p-chloroaniline or p-chloronitrobenzene, subsequently reducing the two nitro groups of the resulting reaction product, if p-chloronitrobenzene is used, to amino groups, and then, if appropriate, the resulting diamine of the general formula I or II is reacted with maleic acid or a derivative thereof to give a bismaleimide of the general formula I or II.

**5.** Use of bismaleimides of the general formula I or II, if appropriate together with diamines of the general formula I or II, according to one of Claims 1 to 3, for the preparation of polymaleimides.

**6.** Polyimides consisting of recurring units which are derived from bismaleimides of the general formula I or II and if appropriate diamines of the general formula I or II, according to one of Claims 1 to 3, the molar ratio of diamine to bismaleimide being 0:1 to 0.8:1.

**7.** Polyimides according to Claim 6, characterised in that they contain diamines and bismaleimides in a molar ratio of 0.2:1 to 0.6:1.

**8.** Polyimides according to Claims 6 or 7, characterised in that they are not completely hardened.

**9.** Polyimides according to one of Claims 6 to 8, characterised in that they are reinforced with fibres.

**10.** Process for the preparation of polyimides according to one of Claims 6 to 9, characterised in that bismaleimides of the general formula I or II are melted at 100 to 300 °C, to form a polyimide resin which if appropriate is hardened completely only in a further process step, and if appropriate bringing the monomer components or the incompletely hardened polyimide resin into contact with reinforcing fibres.

**Revendications**

**1.** Diamines et bismaléimides de formule générale I,

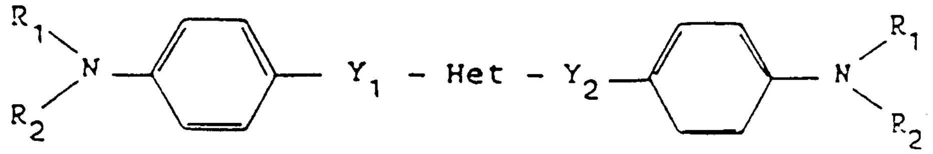

I

dans laquelle $R_1$ et $R_2$ représentent un atome d'hydrogène ou ensemble le groupe -OC-CH = CH-CO-, $Y_1$ et $Y_2$ sont indépendamment l'un de l'autre O ou S, et Het représente un cycle thiazolique ou thiadiazolique.

**2.** Composés de formule II,

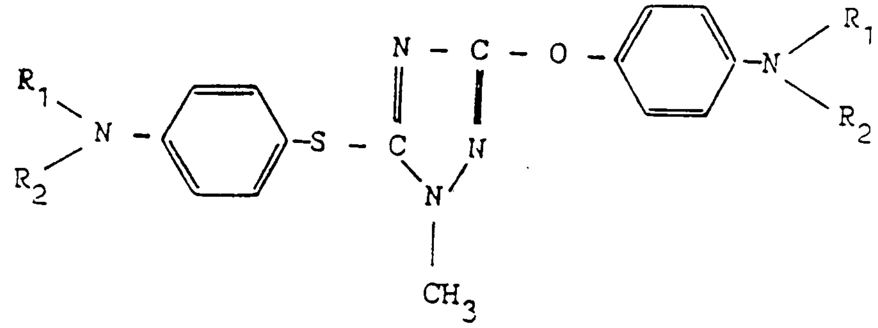

II

dans laquelle $R_1$ et $R_2$ représentent un atome d'hydrogène ou ensemble le groupe -OC-CH = CH-CO-.

**3.** Diamines et bismaléimides selon la revendication 1, caractérisés en ce que $Y_1$ et $Y_2$ sont identiques.

**4.** Procédé pour la production de diamines ou bismaléimides selon l'une quelconque des revendications 1 à 3, caractérisé en ce qu'un composé de formule générale III ou IV :

(K) Na - $Y_1$ - Het - $Y_2$ - Na (K) (III)

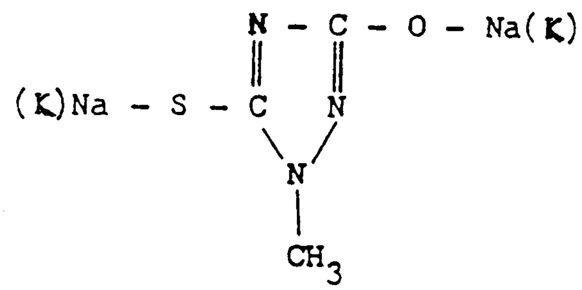

IV

dans lesquelles $Y_1$, $Y_2$ et Het ont la même signification que donné plus haut, est mis en réaction soit avec de la chloroaniline ou du p-chloronitrobenzène, dans lequel les deux groupes nitro du produit réactionnel obtenu sont finalement réduits en groupes amino au moyen de p-chloronitrobenzène, dans lequel on fait réagir les diamines ainsi obtenus de formule générale I ou II, le cas échéant avec de l'acide maléique ou ses dérivés pour obtenir des bismaléimides de formule générale I ou II.

**5.** Utilisation de bismaléimides de formule générale I ou II, le cas échéant associés avec des diamines de formule générale I ou II selon l'une quelconque des revendications 1 à 3, pour la production de polymaléimides.

**6.** Polyimides se composant de motifs qui se répètent et sont issus de bismaléimides de formule générale I ou II et le cas échéant de diamines de formule générale I ou II selon l'une quelconque des revendications 1 à 3, où le rapport molaire de diamine sur le bismaléimide est de 0 : 1 jusqu'à 0,8 : 1.

**7.** Polyimides selon la revendication 6, caractérisés en ce qu'ils contiennent des diamines et des bismaléimides dans un rapport molaire de 0,2 : 1 jusqu'à 0,6 : 1.

**8.** Polyimides selon la revendication 6 ou 7, caractérisés en ce qu'ils ne sont pas entièrement durcis.

**9.** Polyimides selon l'une des revendications 6 à 8, caractérisés en ce qu'ils sont renforcés par des fibres.

**10.** Procédé pour la production de polyimides selon l'une quelconque des revendications 6 à 9, caractérisé en ce que l'on fait fondre des bismaléimides de formule générale I ou II avec ou sans des diamines de formule générale I ou II à une température de 100 jusqu'à 300 ° C, où se forme une résine de polyimide qui, le cas échéant, est complètement durcie seulement dans une étape ultérieure du procédé et en ce que l'on met, le cas échéant, les composants monomères ou la résine de polyimide pas complètement durcie en contact avec les fibres de renforcement.